# EUROPEAN PATENT APPLICATION

(11) **EP 4 685 452 A1**
(43) Date of publication of application: **28.01.2026**
(21) Application number: 24903898.5
(22) Date of filing: 04.09.2024
(51) Int. Cl.: G01N 1/22, G01N 1/24, G01N 33/00, H01M 10/42, G01N 1/00

(54) **GAS COLLECTION DEVICE**

(30) Priority: 15.12.2023 KR 20230183114
(71) Applicant: LG ENERGY SOLUTION, LTD., Seoul 07335 (KR)
(72) Inventor: MOON, Minkook, Daejeon 34122 (KR); KIM, Kyung Min, Daejeon 34122 (KR); PARK, Kwangyeon, Daejeon 34122 (KR); CHOI, Nak Hee, Daejeon 34122 (KR)
(74) Representative: Goddar, Heinz J.
(86) International application number: PCT/KR2024/013312
(87) International publication number: WO 2025/127327

(57) **Abstract**

Disclosed is a gas collecting apparatus comprising: a gas extraction unit configured to extract gas from an analysis target battery; a gas diffusion unit having a gas diffusion space with a variable volume and configured to receive and diffuse the extracted gas from the gas extraction unit; a sampling unit having a gas sampling space configured to receive and sample the diffused gas from the gas diffusion unit; and a control unit configured to control the gas diffusion unit to adjust a volume of the gas diffusion space, wherein a volume of the gas sampling space is fixed.

## Description

### Technical Field

The present application claims the benefit of priority based on Korean Patent Application No. 10-2023-0183114 filed on December 15, 2023, the entire disclosure of which is incorporated as a part of this specification.

The present application relates to an apparatus for collecting gas, and more particularly, to an apparatus for collecting gas capable of sampling gas generated inside a secondary battery by adjusting its concentration to an appropriate level for analysis.

### Background Art

Secondary batteries are batteries that can be used repeatedly through a discharging process that converts chemical energy into electrical energy and a charging process that converts electrical energy into chemical energy, and commonly known types include nickelcadmium (Ni-Cd) batteries, nickel-metal hydride (Ni-MH) batteries, lithium-metal batteries, lithium-ion (Li-ion) batteries, and lithium-ion polymer batteries, etc. Among these secondary batteries, lithium secondary batteries have been commercialized and are widely used due to their high energy density and voltage, long cycle life, and low self-discharge rate.

Various types of gases may be generated inside a lithium secondary battery depending on the charge and discharge reaction, such as hydrogen, oxygen, nitrogen, carbon monoxide, carbon dioxide, hydrocarbons such as CₙH₂ₙ₋₂ (n=2~5), CₙH₂ₙ (n=2~5), and CₙH₂ₙ₊₂ (n=1~5), and other organic gas species.

In addition, lithium secondary batteries generate a large amount of gas due to electrolyte decomposition and degradation in the process of repeated charging and discharging, and this aspect varies depending on the design and usage of the battery. Therefore, it is essential to analyze the gas generated inside the battery to infer a degradation mechanism of the battery during the battery development process.

Therefore, it is very important to collect and accurately analyze the gas generated inside the secondary battery. Information on the composition and content of various gases generated during charging and discharging of lithium secondary batteries is useful for developing battery materials, optimizing the battery manufacturing process, and identifying the cause of battery failure. To this end, the development of technology to collect gas generated inside the secondary battery is important.

The following process may be performed as one of methods for analyzing the gas generated inside the secondary battery.

In order to collect an analysis target gas generated inside the secondary battery, a perforation hole is formed in the case of the secondary battery, and the analysis target gas is extracted through the perforation hole.

The extracted analysis target gas is diffused into a sealed gas diffusion space.

The analysis target gas diffused in the gas diffusion space is sampled in a sampling vessel.

The analysis target gas sampled in the sampling vessel is injected into a gas analysis apparatus such as GC-MS to perform gas analysis.

In the above process, the sensitivity of the gas analysis apparatus is affected by the concentration of the analysis target gas, and if the concentration of the analysis target gas is too low, accurate analysis is difficult. The concentration of the analysis target gas may be determined by the volume of the gas flow path including the gas diffusion space, the sampling vessel, etc. For accurate quantitative analysis, it is necessary to know the exact volume values of the gas diffusion space, the gas flow path, etc., in the entire gas analysis system, but this is not easy. Therefore, a gas collection technology for such a gas analysis system is required.

### Disclosure of the Invention

### Technical Goals

The present disclosure relates to a gas collecting apparatus, and is directed to providing a gas collecting apparatus capable of sampling the analysis target gas generated inside a secondary battery by adjusting the concentration to an appropriate level for analysis.

Technical problems to be achieved by the present disclosure are not limited to the technical problems mentioned above, and other technical problems not mentioned may be clearly understood by those skilled in the art to which the present disclosure pertains from the description below.

### Technical Solutions

A gas collecting apparatus may include:
a gas extraction unit configured to extract gas from an analysis target battery;
a gas diffusion unit having a gas diffusion space configured to receive and diffuse the extracted gas from the gas extraction unit;
a sampling unit having a gas sampling space configured to receive and sample the diffused gas from the gas diffusion unit; and
a control unit configured to control the gas diffusion unit,
wherein a volume of the gas diffusion space may be variable, and
a volume of the gas sampling space may be fixed.

According to an embodiment, the gas extraction unit may include a gas extraction chamber part having a battery accommodation space in which the analysis target battery is accommodated, and a punching part configured to punch a gas extraction hole in the analysis target battery accommodated in the battery accommodation space.

According to an embodiment, the gas diffusion unit may include a base plate part having a plane shape perpendicular to a vertical direction; a cylindrical side wall part which has a lower end part fixed to the base plate part and is capable of being extended or contracted in the vertical direction; a vertical moving part configured to move in the vertical direction, wherein an upper end part of the cylindrical side wall part is fixedly coupled to the moving part; a guide support part configured to guide a movement of the vertical moving part in the vertical direction; and a vertical driver configured to provide a driving force for the vertical moving part configured to move in the vertical direction, wherein a gas diffusion space may be defined by the base plate part, the cylindrical side wall part, and the vertical moving part.

According to an embodiment, a gas port may be located on the base plate part, and the gas may be configured to be injected into or discharged from the gas diffusion space through the gas port.

According to an embodiment, the cylindrical side wall part may have a bellows structure.

According to an embodiment, the vertical moving part may include a body member having a cylindrical shape extending in the vertical direction, and an upper plate member having a plane shape perpendicular to the vertical direction, the upper plate member having a bottom surface fixedly coupled to an upper end part of the body member is fixedly coupled, the upper end part of the cylindrical side wall part may be fixedly coupled to the bottom surface of the upper plate member, the body member may be located inside the cylindrical side wall part, and the gas diffusion space may be defined by a lower end part of the body member, an upper surface of the base plate part, and an inner circumferential surface of the cylindrical side wall part.

According to an embodiment, when the vertical moving part is lowered to a lowest point, the lower end part of the body member may contact the upper surface of the base plate part, and all inner circumferential surfaces of the cylindrical side wall part may face an outer circumferential surface of the body member.

According to an embodiment, the upper plate member may be formed in a disk shape, the guide support part may be formed in a cylindrical shape extending in the vertical direction, an inner diameter of the guide support part may be the same as a diameter of the upper plate member, and the upper plate member may be guided by sliding on an inner circumferential surface of the guide support part.

According to an embodiment, a lower end part of the guide support part may be fixed to the upper surface of the base plate part, a guide hole extending in the vertical direction may be located on a side surface of the guide support part, and the vertical driver may include a power transmission member having a first part received within the guide support part through the guide hole and a second part located outside the guide support part, and wherein the first end part of the power transmission member is coupled to an upper surface of the upper plate member, a vertical moving shaft extending in the vertical direction and coupled to the second end of the power transmission member, and a driving actuator supported on the base plate part, the driving actuator configured to move the vertical moving shaft in the vertical direction.

### Advantageous Effects

A gas collecting apparatus of the present disclosure may be capable of sampling analysis target gas generated inside a secondary battery by adjusting the concentration to an appropriate level for analysis.

The gas collecting apparatus of the present disclosure may control the speed of gas diffusion by adjusting the volume of a gas diffusion space by a driving force acting on a gas diffusion chamber unit when sampling the gas generated inside an analysis target battery, such as a cylindrical or square secondary battery.

The gas collecting apparatus of the present disclosure may, when sampling the analysis target gas diffused in the gas diffusion space of the gas diffusion chamber unit, sample the analysis target gas at an optimized concentration for analysis by a gas analysis unit by varying the volume of the gas diffusion space.

### Brief Description of Drawings

FIG. 1 is a schematic diagram illustrating a gas collecting apparatus according to an embodiment.
FIG. 2 is a side view of a gas diffusion unit according to an embodiment.
FIGS. 3 and 4 are cross-sectional views illustrating a longitudinal section of a gas diffusion unit according to an embodiment.
FIG. 5 is a perspective view illustrating a guide support part according to an embodiment.

### Best Mode for Carrying Out the Invention

Hereinafter, embodiments according to the present disclosure will be described in detail with reference to the accompanying drawings. In this process, the size or shape of the components shown in the drawings may be exaggerated for clarity and convenience of explanation. In addition, terms specifically defined in consideration of the configuration and operation of the present disclosure may vary according to the intentions or customs of users and operators. Definitions of these terms should be made based on the content throughout this specification.

In the description of the present disclosure, it should be noted that the orientation or positional relationship indicated by the terms such as "center", "upper", "lower", "left", "right", "vertical", "horizontal", "inner side", "outer side", "one surface", and "other surface" is based on the orientation or positional relationship shown in the drawing or the orientation or positional relationship normally arranged when using the product of the present disclosure, and it is intended only for explanation and brief description of the present disclosure, and is not to be construed as limiting the present disclosure as it does not suggest or imply that the device or element shown must necessarily be configured or operated in a specific orientation with a specific orientation.

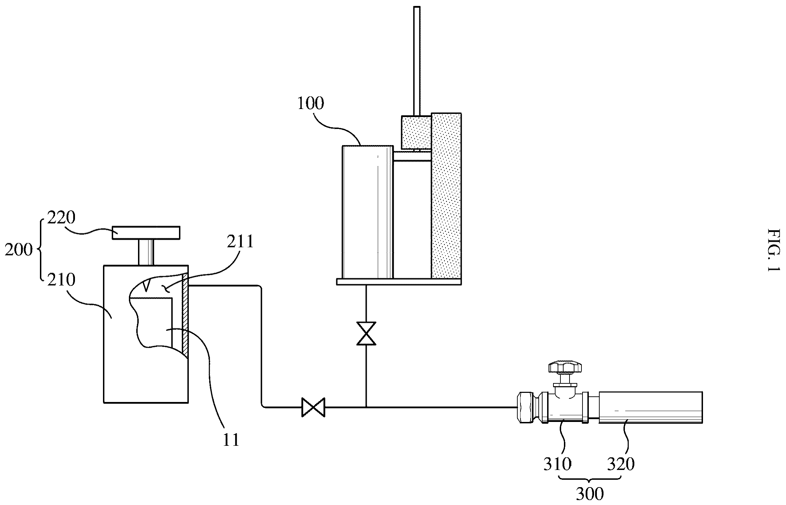

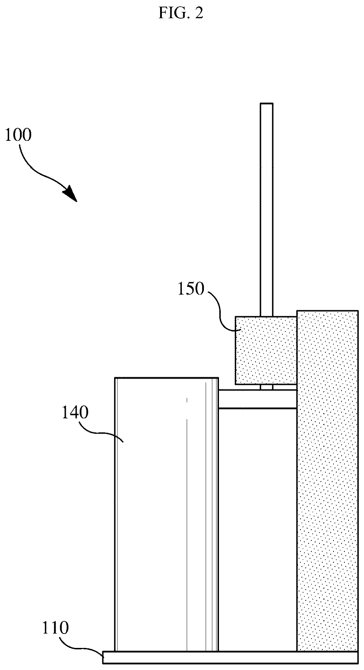

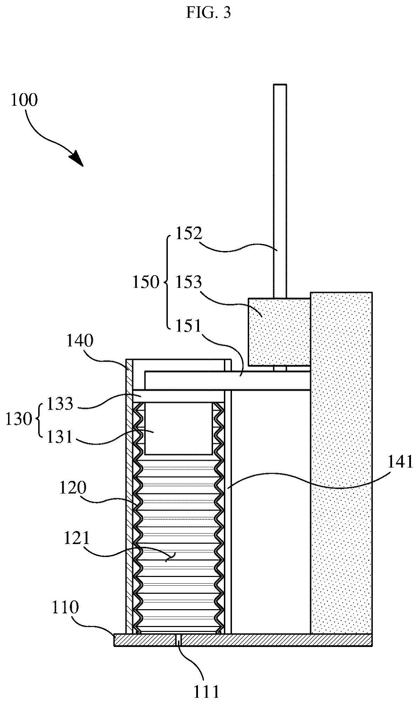

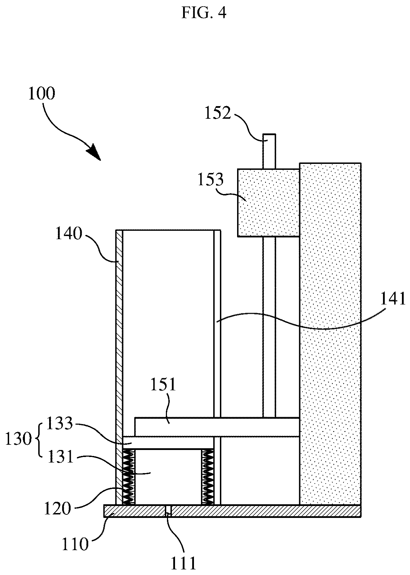

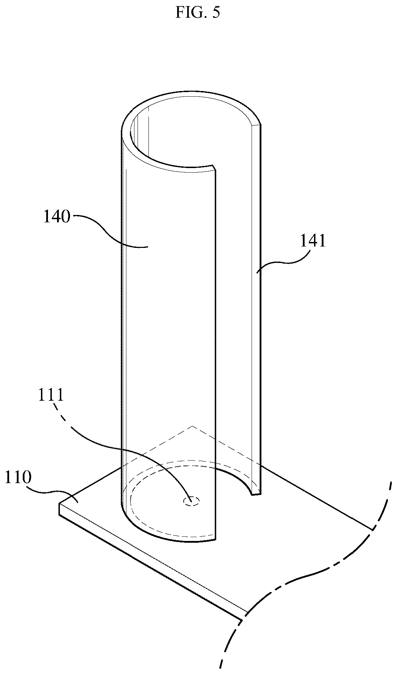

FIG. 1 is a schematic diagram illustrating a gas collecting apparatus according to an embodiment. FIG. 2 is a side view of a gas diffusion unit 100 according to an embodiment. FIGS. 3 and 4 are cross-sectional views illustrating a longitudinal section of the gas diffusion unit 100 according to an embodiment. FIG. 5 is a perspective view illustrating a guide support part 140 according to an embodiment.

Hereinafter, with reference to FIGS. 1 to 5, the gas collecting apparatus of the present disclosure will be described in detail.

The gas collecting apparatus may use a secondary battery having a rigid case, such as a cylindrical secondary battery or a square secondary battery, as an analysis target battery, but is not limited thereto.

The gas collecting apparatus may be for collecting gas generated inside the secondary battery, i.e., gas generated from an active material, a binder, an additive, an electrolyte, etc.

The gas collecting apparatus may include three sealed spaces. Specifically, it may be provided with a battery accommodation space 211, a gas diffusion space 121, and a gas sampling space.

In the gas collecting apparatus, the battery accommodation space 211 may be a space in which the analysis target battery 11 is accommodated. For example, in the battery accommodation space 211, the analysis target battery 11 may have a perforated hole formed on the case. In addition, the analysis target gas generated inside the analysis target battery 11 in the battery accommodation space 211 may be first discharged from the analysis target battery 11, and depending on the situation, the analysis target gas and the electrolyte may be mixed in the battery accommodation space 211.

The gas diffusion space 121 may be a space for adjusting the concentration and pressure of the analysis target gas. The volume of the gas diffusion space 121 may be variable. Specifically, the volume of the gas diffusion space 121 is controlled by a control unit, and the concentration and pressure of the analysis target gas may be adjusted to a state optimized for analysis.

The gas sampling space may be a space that stores the analysis target gas in an optimized state. The gas sampling space may be connected to a gas chromatography-mass spectroscopy (GC-MS), a gas chromatography-pulsed discharge detector (GC-PDD), a gas chromatography-thermal conductivity detector (GC-TCD), a gas chromatography-flame ionization detector (GC-FID), a Fourier transform infrared spectroscopy (FT-IR), and a Raman spectroscopy, etc.

As shown in FIGS. 1 and 2, the gas collecting apparatus of the present disclosure may include:
a gas extraction unit 200 configured to extract the analysis target gas from the inside of the analysis target battery 11;
a gas diffusion unit 100 having the gas diffusion space 121 configured to receive and diffuse the analysis target gas from the gas extraction unit 200;
a sampling unit 300 having the gas sampling space configured to receive and sample the gas diffused in the gas diffusion space 121; and
a control unit (not shown) configured to control the gas diffusion unit 100.

The control unit may be a combination of hardware and software and may be a calculation device for controlling the gas extraction unit 200, the gas diffusion unit 100, and the gas sampling unit 300.

In the gas collecting apparatus, the volume of the gas diffusion space 121 may be variable, and the volumes of the battery accommodation space 211 and the gas sampling space may be fixed.

The gas collecting apparatus may provide the gas diffusion space 121 and the battery accommodation space 211 as separate spaces to prevent the gas from being dissolved again in the electrolyte and improve the accuracy of quantitative analysis.

The gas collecting apparatus may control the gas diffusion speed by changing the volume of the gas diffusion space 121 through the control unit, and may adjust the concentration of the analysis target gas sampled in the gas sampling space.

As illustrated in FIG. 1, the gas extraction unit 200 may include a gas extraction chamber part 210 in which the battery accommodation space 211 where the analysis target battery 11 is accommodated inside is formed, and a punching part 220 that punches a gas extraction hole in the case of the analysis target battery 11 accommodated in the battery accommodation space 211. A jig (not shown) for fixing the analysis target battery 11 in a right position may be provided inside the gas extraction chamber part 210. In addition, the gas extraction chamber part 210 may be equipped with a heater for heating the analysis target battery 11, a chiller for cooling the analysis target battery 11, an impact means or a vibration means for applying a physical force to the analysis target battery 11, a charge/discharge module for charging or discharging the analysis target battery 11, etc.

The punching part 220 may include a punching needle penetrating the case of the analysis target battery 11. The punching needle is located in the battery accommodation space 211, and the punching part 220 may further include a driving means for providing a driving force to the punching needle from the outside of the gas extraction chamber part 210.

As illustrated in FIGS. 2 and 3, the gas diffusion unit 100 may include:
a base plate part 110 formed as a plane perpendicular to a vertical direction;
a cylindrical side wall part 120 which has a lower end part fixed to the base plate part 110 and is capable of being extended or contracted in the vertical direction;
a vertical moving part 130 to which an upper end part of the cylindrical side wall part 120 is fixedly coupled and configured to move in the vertical direction;
a guide support part 140 configured to guide the movement in the vertical direction of the vertical moving part 130; and
a vertical driver 150 configured to provide a driving force for the vertical moving part 130 to move in the vertical direction.

A space surrounded by the base plate part 110, the cylindrical side wall part 120, and the vertical moving part 130 may form the gas diffusion space 121.

The base plate part 110 may be a plate having a plane shape perpendicular to the vertical direction and may be a plate made of a rigid material. For example, the material of the base plate part 110 may be SUS.

A gas port 111 is formed in the base plate part 110, and the analysis target gas may be injected into or discharged from the gas diffusion space 121 through the gas port 111. The center of the gas port 111 may be located at the center of the circular area facing the cylindrical side wall part 120 among the areas of the base plate part 110. A flow path connected to the sampling unit 300 and the gas extraction unit 200 may be connected to the gas port 111. The flow path connected to the gas port 111 may include a hose, a tube, a pipe, etc. As illustrated in FIG. 1, the flow path connected to the gas port 111 may branch off at a specific point and branch off to the sampling unit 300 and the gas extraction unit 200, respectively.

A valve may be provided in the flow path connecting the gas port 111 and the specific point, and the valve may be controlled by the control unit. In addition, a valve may also be provided in the flow path connecting the specific point and the gas extraction unit 200, and the valve may also be controlled by the control unit. As illustrated in FIG. 1, the sampling unit 300 may include a sampling vessel 320 in which the gas sampling space, which is a space in which the analysis target gas is sampled and stored at an optimal pressure and concentration, is formed, and a shut-off valve 310 for opening and closing the sampling vessel 320. The shut-off valve 310 may be connected to the flow path branched from the specific point. The shut-off valve 310 may also be controlled by the control unit.

As illustrated in FIGS. 3 and 4, the cylindrical side wall part 120 may be provided in a cylindrical shape having a vertical central axis. The cylindrical side wall part 120 is capable of being extended or contracted in the vertical direction. The inner diameter value of the cylindrical side wall part 120 may be fixed when extended or contracted in the vertical direction. For example, the cylindrical side wall part 120 may be a bellows structure.

The vertical moving part 130 may include a body member 131 with a cylindrical shape extending in the vertical direction, and an upper plate member 133 formed as a plane perpendicular to the vertical direction and having a bottom surface to which an upper end part of the body member 131 is fixedly coupled.

The upper end part of the cylindrical side wall part 120 may be fixedly coupled to the bottom surface of the upper plate member 133, the body member 131 may be located inside the cylindrical side wall part 120, and a space surrounded by a lower end part of the body member 131, an upper surface of the base plate part 110, and an inner circumferential surface of the cylindrical side wall part 120 may form the gas diffusion space 121. Here, an ideal structure may be a structure in which the inner diameter of the cylindrical side wall part 120 and the outer diameter of the body member 131 are the same, and the coefficient of friction between the outer circumferential surface of the body member 131 and the inner circumferential surface of the cylindrical side wall part 120 may be zero (0).

When the vertical moving part 130 is lowered to the lowest point, the lower end part of the body member 131 may contact the upper surface of the base plate part 110, and all inner circumferential surfaces of the cylindrical side wall part 120 may face the outer circumferential surface of the body member 131. In other words, in the ideal structure, the volume of the gas diffusion space 121 may be zero (0) in the lowest point lowered state of the vertical moving part 130 as shown in FIG. 4.

The upper plate member 133 may be formed in a disk shape, the guide support part 140 may be formed in a cylindrical shape extending in the vertical direction, the inner diameter of the guide support part 140 may be the same as the diameter of the upper plate member 133, and the upper plate member 133 may be guided by sliding on an inner circumferential surface of the guide support part 140. For example, in the ideal state, the coefficient of friction between the side surface of the upper plate member 133 and the inner circumferential surface of the guide support part 140 may be 0. The upper plate member 133 may have a predetermined thickness to prevent the position from being twisted when moving in the vertical direction.

As illustrated in FIG. 5, a lower end part of the guide support part 140 may be fixed to the upper surface of the base plate part 110, a guide hole 141 extending in the vertical direction may be formed on a side surface of the guide support part 140.

The vertical driver 150 may include a power transmission member 151 having one end part inserted into the guide support part 140 through the guide hole 141 and the other end part located outside the guide support part 140, and a vertical moving shaft 152 extending in the vertical direction and coupled to the other end of the power transmission member 151, and a driving actuator 153 that is supported on the base plate part 110 and configured to move the vertical moving shaft 152 in the vertical direction.

The power transmission member 151 may be provided as a support made of a rigid material and in a rod shape extending in a direction perpendicular to the vertical direction.

The power transmission member 151 may be coupled to the upper surface of the upper plate member 133. More specifically, a bottom surface of the one end part of the power transmission member 151 inserted into the guide support part 140 through the guide hole 141 may be attached to the upper surface of the upper plate member 133.

Although the embodiments according to the present disclosure have been described above, they are only illustrative and those skilled in the art will understand that various modifications and embodiments of equivalent range are possible therefrom. Therefore, the true technical protection scope of the present disclosure should be defined by the appended claims.

### <Explanation of Symbols>

11...Analysis target battery 100...Gas diffusion unit 110...Base plate part 111...Gas port 120...Cylindrical side wall part 121...Gas diffusion space 130...Vertical moving part 131...Body member 133...Upper plate member 140...Guide support part 141...Guide hole 150...Vertical driver 151...Power transmission member 152...Vertical moving shaft 153...Driving actuator 200...Gas extraction unit 210...Gas extraction chamber part 211...Battery accommodation space 220...Punching part 300...Sampling unit 310...Shut-off valve 320...Sampling vessel

## Claims

1. A gas collecting apparatus comprising:
a gas extraction unit configured to extract gas from a battery;
a gas diffusion unit having a gas diffusion space configured to receive and diffuse the extracted gas from the gas extraction unit;
a sampling unit having a gas sampling space configured to receive and sample the diffused gas from the gas diffusion unit; and
a control unit configured to control the gas diffusion unit,
wherein a volume of the gas diffusion space is variable, and
a volume of the gas sampling space is fixed.

2. The gas collecting apparatus of claim 1, wherein the gas extraction unit comprises:
a gas extraction chamber part having a battery accommodation space, the battery accommodation space configured to accommodate the battery; and
a punching part configured to punch a gas extraction hole in the battery accommodated in the battery accommodation space.

3. The gas collecting apparatus of claim 2, wherein the gas diffusion unit comprises:
a base plate part having a plane shape perpendicular to a vertical direction;
a cylindrical side wall part having a lower end part fixed to the base plate part, the cylindrical side wall part configured to be extended or contracted in the vertical direction;
a vertical moving part configured to move in the vertical direction, wherein an upper end part of the cylindrical side wall part is fixedly coupled to the moving part;
a guide support part configured to guide a movement of the vertical moving part in the vertical direction; and
a vertical driver configured to provide a driving force for the vertical moving part to move in the vertical direction,
wherein a gas diffusion space is defined by the base plate part, the cylindrical side wall part, and the vertical moving part.

4. The gas collecting apparatus of claim 3, wherein a gas port is located on the base plate part, and
the gas is configured to be injected into or discharged from the gas diffusion space through the gas port.

5. The gas collecting apparatus of claim 3, wherein the cylindrical side wall part has a bellows structure.

6. The gas collecting apparatus of claim 3, wherein the vertical moving part comprises:
a body member having a cylindrical shape extending in the vertical direction; and
an upper plate member having a plane shape perpendicular to the vertical direction, the upper plate member having a bottom surface fixedly coupled to an upper end part of the body member, and
wherein the upper end part of the cylindrical side wall part is fixedly coupled to the bottom surface of the upper plate member,
the body member is located inside the cylindrical side wall part, and
the gas diffusion space is defined by a lower end part of the body member, an upper surface of the base plate part, and an inner circumferential surface of the cylindrical side wall part.

7. The gas collecting apparatus of claim 6, wherein, when the vertical moving part is lowered to a lowest point,
the lower end part of the body member contacts the upper surface of the base plate part, and
all inner circumferential surfaces of the cylindrical side wall part face an outer circumferential surface of the body member.

8. The gas collecting apparatus of claim 6, wherein the upper plate member has a disk shape,
the guide support part has a cylindrical shape extending in the vertical direction,
an inner diameter of the guide support part is the same as a diameter of the upper plate member, and
the upper plate member is configured to be guided by sliding on an inner circumferential surface of the guide support part.

9. The gas collecting apparatus of claim 8, wherein a lower end part of the guide support part is fixed to the upper surface of the base plate part,
a guide hole extending in the vertical direction is located on a side surface of the guide support part, and
the vertical driver comprises:
a power transmission member having a first part received within the guide support part through the guide hole and a second end part located outside the guide support part, wherein the first end part of the power transmission member is coupled to an upper surface of the upper plate member;
a vertical moving shaft extending in the vertical direction and coupled to the second end part of the power transmission member; and
a driving actuator supported on the base plate part, the driving actuator configured to move the vertical moving shaft in the vertical direction.
